# EUROPEAN PATENT APPLICATION

(11) **EP 3 733 124 A1**
(43) Date of publication of application: **04.11.2020**
(21) Application number: 18896530.5
(22) Date of filing: 27.12.2018
(51) Int. Cl.: A61F 2/07

(54) **AORTIC STENT GRAFT CAPABLE OF STEP-BY-STEP RELEASE**

(30) Priority: 29.12.2017 CN 201711483955
(71) Applicant: Hangzhou Endonom Medtech Co., Ltd, Binjiang District Hangzhou Zhejiang 310052 (CN)
(72) Inventor: GUO, Wei, Hangzhou, Zhejiang 310052 (CN); WANG, Yongsheng, Hangzhou, Zhejiang 310052 (CN); LI, Anwei, Hangzhou, Zhejiang 310052 (CN)
(74) Representative: Hamer, Christopher K.
(86) International application number: PCT/CN2018/124416
(87) International publication number: WO 2019/129153

(57) **Abstract**

The present invention discloses a staged release aortic stent graft, comprising a tubular covering and annular support frames, connectors for a release guide wire to pass through, are arranged axially from the proximal end to the distal end on the covering, and at least two columns of the connectors are arranged axially with spacing. The invention provides a staged release aortic stent graft which can be precisely positioned during release and has high stability during assembly.

## Description

### TECHNICAL FIELD

The invention relates to the technical field of medical devices, and particularly relates to a stent graft, in particular to a staged release aortic stent graft used for interventional treatment of aortic diseases.

### BACKGROUND

Aortic aneurysm refers to the locally or diffusely abnormal expansion of the aortic wall, which compresses the surrounding organs and causes symptoms. According to structure, aortic aneurysms can be divided into true aortic aneurysms, false aortic aneurysms, and dissecting aortic aneurysms. Aortic aneurysms cause an increase in the medial pressure of the blood vessels, so they are progressively enlarged. If they develop for a long time, they eventually rupture, and the larger the tumor, the greater the possibility of rupture. According to statistics, without surgical treatment, 90% of those who have thoracic aortic aneurysms die within 5 years, and 3/4 of those who have abdominal aortic aneurysms die within 5 years.

Thoracic aortic endovascular repair is currently used to treat aortic ectasia lesions such as aortic dissection, aortic penetrating ulcer, aortic intermural hematoma, thoracic aortic aneurysm, and pseudoaneurysm. And since the first case of abdominal aortic endovascular repair has been used in the treatment of abdominal aortic aneurysms in the 1990s, because of its advantages of low trauma, short operation and hospital stay, fast postoperative recovery, low perioperative mortality and complication rate, etc., it is rapidly developing in just 20 years.

Aortic lumen repair often uses an expandable stent graft as a treatment device. In order to maintain good adherence to the blood vessel to be repaired, the diameter of the stent graft after release is generally greater than about 10% of the diameter of the blood vessel and completely released stent graft is adhered to the blood vessel, so it cannot be readjusted even when the release position is imprecise, which requires the operator to have extensive experience and spends more time to accurately locate the angle of release of the stent before the stent is released, thus we need to develop a stent that can adjust its position when the stent is released in the blood vessel.

### SUMMARY

The technical problem to be solved by the present invention is to provide a staged release aortic stent graft which can be accurately positioned during release and has high stability during assembly in view of the defects of the prior art.

The technical solution adopted by the present invention to solve its technical problems is:

A staged release aortic stent graft, comprising a tubular covering and annular support frames, and the connectors for a release guide wire to pass through are arranged axially from the proximal end to the distal end on the covering, and at least two columns of the connectors are arranged circumferentially with spacing.

Further, in the staged release aortic stent graft, preferably the connector is a closed-loop structure or an open-loop structure for the release guide wire to pass through or wind around; the closed-loop structure is a through hole on the connector for the release guide wire to pass through, or a through hole or a gap surrounded by the connector cooperated with the covering for the release guide wire to pass through, and the open-loop structure has a limit groove for the release guide wire to pass through.

Further, in the staged release aortic stent graft, preferably the connector is a flexible connection buckle arranged on the covering or a flexible connection buckle that can be attached and fixed on the covering.

Further, in the staged release aortic stent graft, preferably the connector is a coil fixed on the outer wall surface of the covering; or, the connector is a piece of wire fixed axially with spacing, and a gap for the release guide wire to pass through is formed between the fixed wire with spacing and the covering; or, the connector is a through hole formed in the covering; or, the connectors are at least two columns of flexible connection buckles with the limit groove, and the openings of the limit grooves in different columns are arranged in the opposite direction.

Further, in the staged release aortic stent graft, preferably a plurality of axially arranged support rods are fixed with spacing at least at the proximal end along the circumferential direction on the covering.

Further, in the staged release aortic stent graft, preferably the support rod is fixed on the outer wall or the inner lumen wall surface of the covering, and the connector is fixed on the outer wall surface of covering that corresponding to the support rod, or the connector is arranged on the support rod or the covering around the support rod.

Further, in the staged release aortic stent graft, preferably the support rod is fixed on the covering by sewing, heat sealing or bonding.

Further, in the staged release aortic stent graft, preferably at least one fixing point for fixed connection with the covering is arranged on the support rod, and the fixing point is a connection hole or an opening slot with an opening arranged on the support rod.

Further, in the staged release aortic stent graft, preferably the support rods are arranged parallel to the central axis of the stent; or the support rods are arranged with each other in a shape of a figure eight expressed in Simplified Chinese or a shape of an inverted figure eight expressed in Simplified Chinese.

Further, in the staged release aortic stent graft, preferably the stent is a tubular structure extending with an equal diameter or a non-equal diameter.

Further, in the staged release aortic stent graft, preferably a fenestration for arranging branch stents or branch blood vessels is arranged at the proximal end or middle of the stent.

Further, in the staged release aortic stent graft, preferably the connectors are arranged at both sides of the fenestration, for restraining a portion of the stent behind the fenestration.

Further, in the staged release aortic stent graft, preferably the stent is a tubular structure extending with a non-equal diameter, which comprises a main body section and an extension section, and the diameter of the main body section is greater than the diameter of the extension section, and a transition section is arranged between the main body section and the extension section; a fenestration for placing branch stents or branch blood vessels is arranged at the main body section or/and the transition section.

Further, in the staged release aortic stent graft, preferably the connectors are arranged at both sides of the fenestration of the main body section and the transition section, for restraining a portion of the stent behind the fenestration.

Further, in the staged release aortic stent graft, preferably a plurality of axially arranged support rods are fixed with spacing at least at the proximal end along the circumferential direction on the covering, and the support rod is fixed on the outer wall or the inner lumen wall surface of the covering, and the connectors are fixed on outer wall surface of covering that corresponding to the support rods, or the connectors are arranged on the support rods or the covering around the support rods.

The invention provides a stent graft, which is provided with a plurality of connectors on the covering, and the stent is folded between the two columns of connectors, that is two or more adjacent connectors are drawn closer, and a release guide wire of the delivery device passes through the connectors; after the restraint, the stent can maintain a semi-deployed state in the blood vessel to be repaired, and the diameter of the stent in semi-deployed state is smaller than the diameter of blood vessel, and can freely rotate and move longitudinally in the blood vessel, facilitating a precise position during the release process.

There are support rods inside or outside the covering, on the one hand, the support rods can facilitate the folding of the stent, on the other hand, the support rods can also be used as a stud during the assembly of the stent, which can not only ensure the stability of the stent during assembly, but also ensure a semi-deployed state with stable circumferential structure after the stent is partially released.

### BRIEF DESCRIPTION OF THE ACCOMPANYING DRAWINGS

The present invention will be further described below with reference to the drawings and embodiments. In the drawings:
Fig. 1 is a schematic structural view of a stent graft in Embodiment 1 of the present invention;
Fig. 2 is a schematic rear structural view of the semi-deployed stent graft under the restraint according to Embodiment 1 of the present invention.
Fig. 3 is a schematic structural view of single-ring annular support frame according to Embodiment 1 of the present invention;
Fig. 4 is a schematic diagram of the partial release of the only proximal covering during the release of the stent graft according to Example 1 of the present invention;
Fig. 5 is a schematic structural view of a stent graft in Embodiment 2 of the present invention;
Fig. 6 is a schematic structural view of a support rod according to Embodiment 2 of the present invention;
Fig. 7 is a schematic rear structural view of the semi-deployed stent graft under the restraint according to Embodiment 2 of the present invention.
Fig. 8 is a schematic structural view of a stent graft in Embodiment 3;
Fig. 9 is a schematic structural view of the funnel-shaped annular support frame according to Embodiment 3;
Fig. 10 is a schematic structural view of a stent graft in Embodiment 4;
Fig. 11 is a schematic structural view of a stent graft in Embodiment 5;
Fig. 12 is a schematic structural view of the fenestration support frame in Embodiment 5;
Fig. 13A is a schematic rear structural view of partially release of only the proximal covering during the release of the stent graft according to the embodiment 5.
Fig. 13B is a schematic front structural view of partially release of only the proximal covering during the release of the stent graft according to the embodiment 5.

### DETAILED DESCRIPTION OF ILLUSTRATED EMBODIMENTS

In order to have a clearer understanding of the technical features, purposes and effects of the present invention, the specific embodiments of the present invention will now be described in detail with reference to the drawings.

In the present invention, the "proximal end" refers to the portion of the stent or stent component that near the patient's heart along the direction of blood flow, and the "distal end" refers to the portion of the stent or stent component that away from the patient's heart.

Front and rear definition: tubular surface of the stent with the fenestration is the front wall, and the tubular surface of the stent opposite the fenestration is the rear wall, and the tubular surfaces on both sides of the stent between the front and rear walls are the side walls.

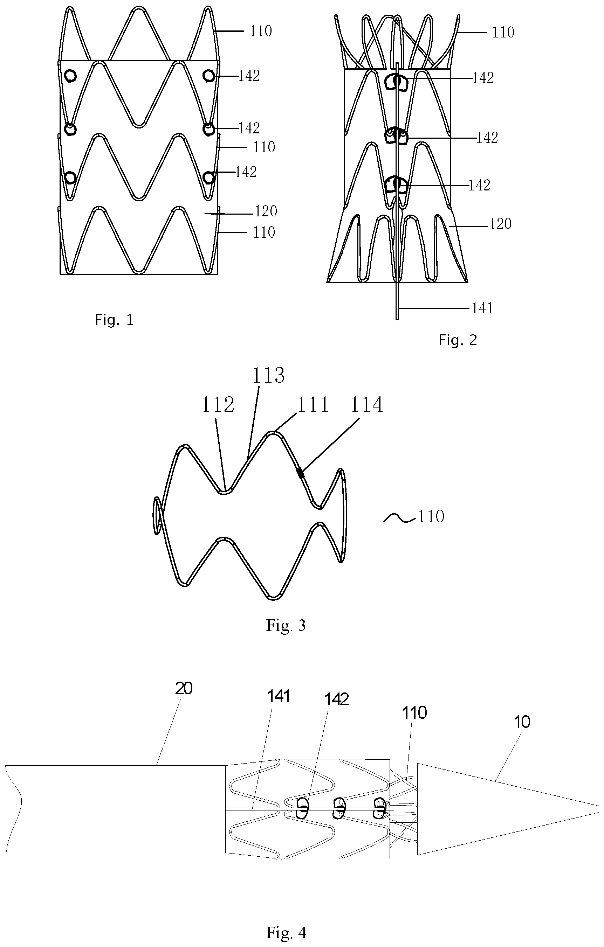

In Embodiment 1, as shown in Fig. 1, a staged release aortic stent graft comprises a tubular covering 120 and annular support frames 110, and the connectors 142 for the release guide wire to pass through are arranged axially from the proximal end to the distal end on tubular covering 120, and at least two columns of the connectors 142 is arranged axially with spacing.

The shape of the tubular covering 120 may be a straight cylindrical shape with equal diameter, or a funnel shape, a dumbbell shape, of a non-isodiametric structure, or other suitable shapes. In this embodiment has a straight cylindrical shape with an isodiametric structure. The covering 120 is made of a polymer material with good biocompatibility. In this embodiment, a PET film is preferred, and the film thickness is 0.07 mm to 0.1 mm. The covering 120 has good mechanical strength and anti-endoleak performance.

The annular support frame 110 plays a role of supporting the covering 120. A plurality of annular support frames 110 are sequentially sewn on the covering 120 in parallel along the axial direction of the covering 120 with uniform spacing. The annular support frame 110 on the proximal end and/or the distal end of the stent is a bare stent partially sewn on the covering 120. As shown in Fig. 1, a plurality of annular support frames 110 are arranged in sequence from the proximal end to the distal end of the covering 120. The annular support frames 110 located at the proximal end of the stent graft is a semi-sewn stent, and its extension section is sewn on the covering 120 separately. As shown in Fig. 3, each annular support frame 110 is in the shape of a tube, and is composed of multiple Z-shaped or sine waves. Each Z-shaped or sine wave has one peak 111 and one adjacent trough 112, and there is a connecting rod 113 between the peak 111 and the adjacent trough 112. Each annular support frame 110 is woven from a super-elastic nickel-titanium wire. The diameter of the nickel-titanium wire is relatively thin, which can be between 0.2 and 0.5 mm. In this embodiment, it is woven from a nickel-titanium wire with a diameter of 0.45 mm. The number of Z-shaped or sine waves is six, and the vertical height of the annular support stent is 12 mm. As shown in Fig. 3, there is one connecting steel sleeve 114 on each annular support frame 110. The two ends of the nickel-titanium wire are inside the connecting steel sleeve 114, and then the two ends of the nickel-titanium wire are fixed inside the steel sleeve by mechanical compaction or weld. The annular support frames 110 are fixed to the inner surface or the outer surface of the covering 120 by other processes such as sewing, heat sealing or bonding; the stent in this embodiment is specification 30, which means, the diameter of the annular support frame 110 and the covering 120 are both 30 mm. The annular support frames 110 are fixed on the outer surface of the covering 120 by using a sewing process, so as to keep the whole stent graft with good resilience.

The connectors 142 are used for the release guide wire 141 to pass through axially, and serves to assist the release guide wire 141 to restrain the stent in the radial direction to form a semi-deployed state, as shown in Fig. 2, which is a schematic rear structural view of the semi-deployed stent graft of the Embodiment 1 in present invention under the restraint by the release guide wire passing through the connectors. The structure of the connectors 142 needs to meet the above purpose at the same time, so the connectors 142 can choose two kinds of structure: a closed-loop structure or an open-loop structure for the release guide wire 141 to pass through or wind around; in the first structure, the closed-loop structure means that the connectors 142 has a through hole through which the release guide wire 141 passes, or the connectors 142 cooperates with the covering to surround to arrange a through hole or gap through which the release guide wire 141 passes. Specifically, the first embodiment is: the connector 142 is a coil fixed on the outer wall surface of the covering; the second embodiment is: the connector 142 is a piece of wire fixed axially with spacing, and a gap is formed between the fixed wire with spacing and the covering for the release guide wire 141 to pass through; the third embodiment is: the connector 142 is a through hole directly formed in the covering.

The open-loop structure is arranged with a limit groove through which the release guide wire 141 passes, that is, the connectors 142 are at least two columns of flexible connection buckles with a limit groove, and the openings of the limit grooves in different columns are arranged in the opposite direction. The release guide wire 141 successively bypasses the limiting grooves of different columns of flexible connection buckles to restrain the stent.

In order to prevent the connector from damaging the blood vessel during the release of the stent, the connector 142 is a flexible connection buckle provided on the covering or can be attached and fixed on the covering, preferably made of a biocompatible polymer material, such as polyester, and it can also choose metal material, such as metal wire.

In this embodiment, the connector 142 is a coil with a through hole, and the release guide wire 141 with a wire diameter of 0.5 mm to 1.5 mm can pass through the through hole. In this embodiment, the material of the release guide wire 141 is preferably nickel-titanium alloy wire, and the wire diameter is 0.5 mm, and the number of connectors 142 is six, and they are uniformly fixed on the covering 120 in two columns, wherein each column has three connectors 142, and those three connectors 142 are arranged axially and spaced apart. As shown in Fig. 2, when assembling, first pull the two columns of connectors 142 together, and the fixed release guide wire 141 of the delivery device respectively passes through the different columns of connectors 142 in sequence to form a restraint to the stent. Under the restraint of release guide wire 141, the stent graft is maintained as a semi-deployed state, and then the stent is fully clamped in the sheath of the delivery device. As shown in Fig. 4, when releasing, first release the portion of the proximal end of the stent graft that is not restrained by the release guide wire 141, at this time, the covering 120 between the connectors 142 is still in a restrained state, and the distal end of covering 120 is bundled in the outer sheath 20, and the annular support frame 110 as the bare stent at the proximal end is still restrained in TIP head 10 of the delivery device, the stent graft is not completely released, whose overall diameter is smaller than the diameter of the blood vessel, so that it can be freely rotated and moved longitudinally in the blood vessel, facilitating for precise positioning of the stent. After finding the correct release position of the stent, the release guide wire 141 is withdrawn, and the stent graft is completely released and firmly fits in the blood vessel.

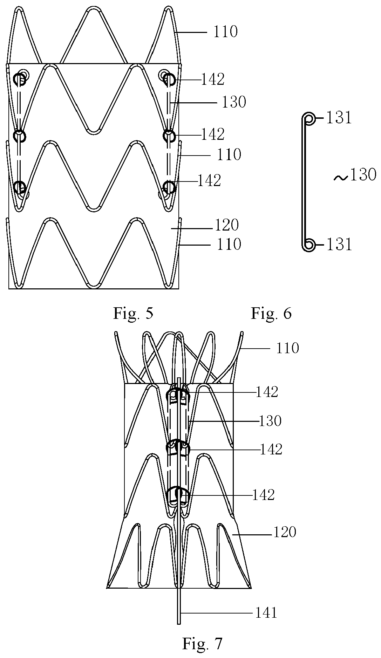

The Embodiment 2, as shown in Fig. 5, this embodiment is an improvement based on Embodiment 1.

A staged release aortic stent graft comprises a tubular covering 120 and annular support frames 110 fixed on the covering 120. A plurality of axially arranged support rods 130 are fixed at least at the proximal end along the circumferential direction of covering 120 on the surface of the covering 120, preferably the inner lumen wall surface, and connectors 142 for fixing the release guide wire are arranged on outer side of the covering 120 corresponding to the support rod 130.

The first function of the support rod 130, that is, the most important function is to form a stent in a semi-deployed state, that is, two or more adjacent support rods 130 are drawn close to each other parallel and fixed by connectors 142, during the drawing process, the support rod 130 can always maintain the flatness of the covering and the stability of the overall structure of the stent. The covering 120 and the annular support frames 110 between the support rods 130 are folded to reduce the diameter of the stent, forming a semi-deployed state, and the diameter of stent of semi-deployed state is smaller than the diameter of the blood vessel, so that it can rotate freely and move longitudinally in the blood vessel to adjust the position, facilitating precise positioning of the stent during the release process.

The support rods 130 are arranged along the axis of the covering 120 in the circumferential direction of the covering 120. The plurality of support rods 130 are preferably arranged in an axial symmetry. There are two types of positional relationship between the support rods 130: one is the support rods 130 are arranged parallel to the central axis of the stent; the other is non-parallel to the central axis of the stent, that is, the support rods 130 are arranged in a shape of a figure eight expressed in Simplified Chinese or a shape of an inverted figure eight expressed in Simplified Chinese. As shown in Fig. 7, after restraining and fixing, the support rod 130 arranged parallel to the central axis of the stent, the diameter of the stent is reduced at the same proportion. For example, the original is a straight cylindrical stent, and the semi-deployed state is also straight cylindrical. After restraining and fixing, the support rod 130 that is not arranged parallel to the central axis of the stent, and the diameter of the stent decreases at the different proportion, for example, the original is a straight cylindrical stent, and the semi-deployed state is a cone. In this embodiment, the support rod 130 is preferably arranged parallel to the central axis of the stent. The number of support rods 130 is generally 2-6, preferably 2-3.

The support rod 130 is arranged at least at the proximal end of the covering 120, which means that the proximal end of the support rod 130 is arranged at the proximal end of the covering 120. The support rod 130 can extend toward the distal end of the stent, and its extension length can be selected as needed. It can be extended to the middle of the axial direction of the stent, and it also can be extended to the distal end of the axial direction of the stent.

As shown in Fig. 5, in this embodiment, it is preferable that two support rods 130 are fixed on the inner surface of the cylindrical structure of the covering 120, and the support rods 130 is positioned at the rear of the proximal end of the covering 120, and they are fixed on the inner wall of the covering 120 by the processes such as sewing, heat sealing or bonding, preferably by sewing.

The material of the support rod 130 is metal or polymer material with a certain support strength, preferably metal material, such as nickel-titanium alloy wire, with wire diameter ranging from 0.3 mm to 0.6 mm. In this embodiment, the wire diameter is preferably 0.45 mm. The structure of the support rod 130 is shown in Fig. 6, and the support rod 130 shown in the figure is a straight rod structure. At least one fixing point is arranged on the support rod 130, and the support rod 130 is fixed on the inner wall of the covering 120 by sewing, heat sealing or bonding. The fixing point is a connection hole or an opening slot with an opening arranged on the support rod 130. In this embodiment, there are two fixing points on both ends of the support rod 130 respectively. The fixing points in this embodiment are two limit rings 131 with connection holes, which are formed by curling the two ends of the support rod 130, and the diameter of the limit ring 131 ranges from 1.5 mm to 3.5 mm. In this embodiment, the diameter of the limit ring 131 is preferably 2.5 mm. The limit ring 131 is fixed to the proximal end of the covering 120 by sewing to prevent the support rod 130 from sliding off along the axial direction of the stent. Besides the connection hole, the fixing point can also be a non-closed-loop structure, or an opening slot with an opening, for example: bending somewhere in the support rod 130 to form a semi-circular or arc-shaped opening slot, and the support rod 130 cannot move axially after fixed at the opening slot.

As shown in Fig. 7, in this embodiment, the support rod 130 plays a supporting role in the axial direction, and also serves as a stud during assembly of the stent graft 100, which can not only ensure the stability of the stent during assembly, but also ensure a semi-deployed state with stable circumferential structure after the stent is partially released, so that the diameter of the stent in the semi-deployed state is smaller than the diameter of the blood vessel, and the position can be adjusted by free rotation and longitudinal movement in the blood vessel, facilitating an precise positioning of the stent during the release process.

The rest of the structure is the same as that in Embodiment 1, and will not be repeated here.

The Embodiment 3, this embodiment is an improvement based on Embodiment 2. Difference between the two of them: the covering 120 of the present embodiment is a tubular structure with non-equal diameter extending.

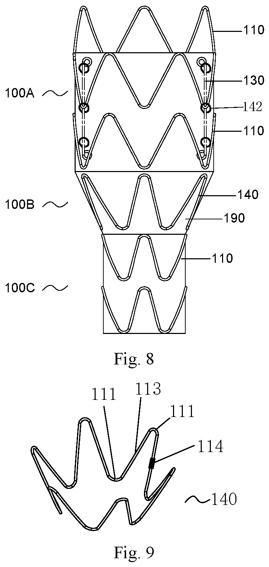

As shown in Fig. 8, the stent graft of Embodiment 2 of the present invention comprises the multi-rings annular support frames 110. It comprises a funnel-shaped covering 120 and annular support frames 110 and 140 fixed on the covering 120, and two support rods 130 are fixedly arranged on the inner lumen wall surface of the covering 120. The arrangement of the support rods 130 of Embodiment 2 are the same as the support rods 130 of Embodiment 1, and will not be repeated here.

The stent graft has a funnel-shaped structure and comprises a main body section 100A and an extension section 100C. A transition section 100B is arranged between the main body section 100A and the extension section 100C, and the diameter of the extension section 100C is smaller than the diameter of the main body section 100A. Both the main body section 100A and the extension section 100C comprise a straight cylindrical covering 120 and annular support frames 110, respectively, and the transition section 100B includes a frustum-shaped covering 190 and a funnel-shaped annular support frame 140.

As shown in Fig. 9, the funnel-shaped annular support frame 140 is composed of multiple Z-shaped or sine waves. Each Z-shaped or sine wave has one peak 111 and one adjacent trough 112, and there is a connecting rod 113 between the peak 111 and the adjacent trough 112. The difference between the annular support frame 140 and the annular support frame 110 in the embodiment is that the annular support frame 140 has a taper, and the difference of the diameter between the two ends of the annular support frame 140 is 10 mm.

The rest of the structure is the same as that in Embodiment 2, and will not be repeated here.

The Embodiment 4, this embodiment is an improvement based on Embodiment 2.

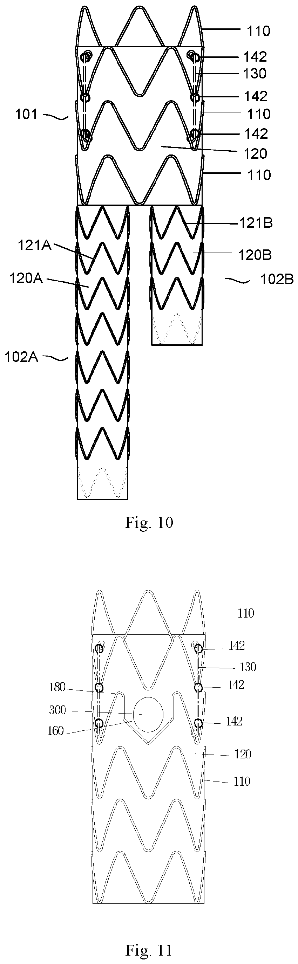

The difference between the two of them: the stent graft of this embodiment is arranged with a long branch section 102A and a short branch section 102B at the distal end of the stent graft of Embodiment 2. That is, as shown in Fig. 10, the stent graft of Embodiment 2 is the main body stent 101 of Embodiment 4. The arrangement of the main body stent 101 is the same as that of Embodiment 2, and two support rods 130 are fixedly arranged on the inner lumen wall surface of the covering 120 and connectors 142 for fixing the release guide wire are arranged on outer side of the covering 120 corresponding to the support rods 130. The arrangement of the support rods 130 and support rods 130 of Embodiment 4 is the same as that of Embodiment 2, and will not be repeated here.

The distal end of the main body stent 101 is connected with two branch sections, which respectively are a long branch section 102A and a short branch section 102B.

The long branch section 102A and the short branch section 102B are respectively arranged with branch section covering 120A and branch section covering 120B in the circumferential direction, and branch section covering 120A and branch section covering 120B are sewn together with the covering 120 of the main body stent 101 to form as a whole structure, or an integral structure integrally formed with the covering 120. The branch section covering 120A has a straight cylindrical structure with a diameter ranging from 10 mm to 14 mm and a length of 70 mm; the branch section covering 120B also has a straight cylindrical structure with a diameter ranging from 10 mm to 14 mm and a length of 30 mm. A transition zone may also be arranged between the covering 120 and the branch section covering 120A and the branch section covering 120B, for connecting the main body stent to the long branch section 102A and the short branch section 102B. The length of the transition zone ranges from 10 mm to 20 mm, preferably 15mm. The materials of the covering 120, the branch section covering 120A and the branch section covering 120B can be selected from polyester, polyurethane, ePTFE, PET or other polymer materials. In this embodiment, the material is PET film, and the thickness of the PET film ranges from 0.07mm to 0.12mm, preferably 0.1mm.

The outer wall or inner wall of the branch section covering 120A and the branch section covering 120B are respectively fixed by varying amounts of branch annular stent 121A and branch annular stent 121B, which are sequentially spaced apart and sewn separately. The stent material of the branch annular stent 121A and branch annular stent 121B is preferably a nickel-titanium alloy wire with good biocompatibility and super-elasticity.

The rest of the structure is the same as that in Embodiment 2, and will not be repeated here.

The Embodiment 5, this embodiment is an improvement based on Embodiment 2 or Embodiment 3. The difference is that a fenestration 300 for arranging a branch blood vessel is disposed on the covering 120.

The covering 120 has two kinds of structure, and there are also two situations for disposing the fenestration 300. As shown in Fig. 11, one is the covering 120 is a tubular structure extending with an equal diameter, a fenestration 300 for arranging branch blood vessels is arranged at the proximal end or middle of the covering 120; the fenestration 300 is positioned on the covering 120 between two adjacent support rods, and the fenestration support frame 180 surrounding the fenestration is arranged away from the fenestration 300. The two or more fenestrations 300 are simultaneously arranged in the same area between the two support rods 130, preferably on the front wall of the stent, and the centerlines of the two fenestrations are on the same axis. The other one is that the covering 120 is a tubular structure extending with a non-equal diameter, which comprises a main body section and an extension section. The diameter of the main body section is greater than the diameter of the extension section, and a transition section is arranged between the main body section and the extension section; a fenestration 300 for arranging the branch blood vessel is arranged at the main body section or the transition section.

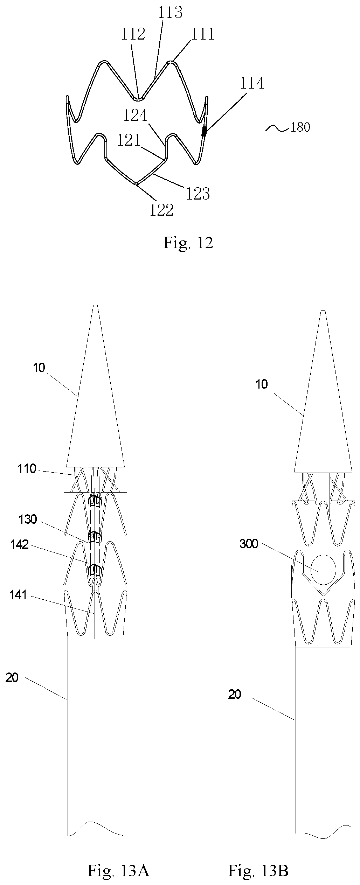

As shown in Fig. 12, the present invention is an improvement based on Embodiment 2. The annular support frames comprise annular support frames 110 as a main body, and a fenestration support frame 180 arranged corresponding to the fenestration 300. In the fenestration support frame 180, fenestration waveform units of fenestration 300 is arranged corresponding to the fenestration 300. That is, in the stent graft the annular support frames 110 of this embodiment comprise annular support frames 110 with multi-rings structure and a uniform diameter, and a fenestration support frame 180, a single-ring metal ring 160 arranged at the fenestration 300, and two support rods fixedly arranged at the inner surface of the covering 120. As shown in Fig. 12, fenestration support frame 180 is composed of multiple Z-shaped or sine waves and a fenestration waveform unit. Each Z-shaped or sine wave has one peak 111 and one adjacent trough 112, and there is a connecting rod 113 between the peak 111 and the adjacent trough 112; the fenestration waveform unit has two peak 121 and one trough 122, and is located between two peaks 111 of a Z-shaped or sine wave. There are two connecting rods 123 the peak 121 and the trough 122 of the fenestration waveform unit, and there are two connecting rods 124 the peak 121 and two peaks 111 of the Z-shaped or sine wave. The fenestration support frame 180 is woven from a piece of superelastic nickel-titanium wire with a wire diameter ranging from 0.2 to 0.5 mm, preferably 0.45 mm in this embodiment. The number of Z-shaped or sine waves is six, and the vertical height of the fenestration support frame 180 is 15 mm. There is one connecting steel sleeve 314 on the fenestration support frame 180. The two ends of the nickel-titanium wire are inside the connecting steel sleeve 114, and then the two ends of the nickel-titanium wire are fixed inside the steel sleeve by mechanical compaction or weld.

As shown in Fig. 13A, the stent graft maintains a semi-deployed state under the restraint of the fixed release guide wire 141 on the delivery device, and the release guide wire 141 passes through the connectors 142 in sequence. In this embodiment, the material of the release guide wire 141 is preferably nickel-titanium alloy wire, and the wire diameter is 0.5 mm, and the number of connectors 142 is six, and they are fixed on the support rods 130 in two columns, wherein each column has three connectors 142, and those three connectors 142 are located at both ends and in the middle of the support rods 130.

As shown in Fig. 13A-13B, when the stent graft is released, it is in a semi-deployed state under the restraint of the guide wire 141, and a portion of the proximal end of the stent that behind the fenestration 300 is still in a restrained state, and the overall diameter of the stent is smaller, and front annular fenestration 300 is in the deployed state, which in the clinical application process, because the stent graft is not fully released, the distal covering is bundled in the outer sheath 20 and the proximal bare stent 110 is still retrained in the TIP head 10 of the delivery device, and it can be conveniently rotated axially and circumferentially in the blood vessel through the delivery system thanks to its smaller overall diameter, which can more quickly and precisely locate the precise position of the annular fenestration 300, so that it leads to smoothly anastomose with the branch blood vessel, shortened operation time, and an improvement of the success rate of surgery. On the other hand, the support rod 130 can also be used as a stud during the assembly of the stent graft, which can not only ensure the stability of the stent during assembly, but also ensure a semi-deployed state with stable circumferential structure after the stent is partially released, preventing the fenestration 300 from deformity when the stent is in a semi-assembled or semi-deployed state, which results in an imprecise positioning.

The rest of the structure is the same as that in Embodiment 2 or 3, and will not be repeated here.

The above-mentioned embodiments only express several implementations of the present invention, and their descriptions are more specific and detailed, but they should not be construed as limiting the patent scope of the present invention. It should be noted that, for a person of ordinary skill in the art, without departing from the concept of the present invention, several modifications and improvements can also be made, which all fall within the protection scope of the present invention.

## Claims

1. A staged release aortic stent graft, comprising a tubular covering and annular support frames, wherein connectors for a release guide wire to pass through are arranged axially from the proximal end to the distal end on the covering, and at least two columns of the connectors are arranged axially with spacing.

2. The staged release aortic stent graft according to claim 1, wherein the connector is a closed-loop structure or an open-loop structure for the release guide wire to pass through or wind around; the closed-loop structure is a through hole on the connector for the release guide wire to pass through, or a through hole or a gap surrounded by the connector cooperated with the covering for the release guide wire to pass through; the open-loop structure has a limit groove for the release guide wire to pass through.

3. The staged release aortic stent graft according to claim 2, wherein the connector is a flexible connection buckle arranged on the covering or a flexible connection buckle that can be attached and fixed on the covering.

4. The staged release aortic stent graft according to claim 3, wherein the connector is a coil fixed on the outer wall surface of the covering; or, the connector is a piece of wire fixed axially with spacing, and a gap for the release guide wire to pass through is formed between the fixed wire with spacing and the covering; or, the connector is a through hole formed in the covering; or, the connectors are at least two columns of flexible connection buckles with the limit groove, and the openings of the limit grooves in different columns are arranged in the opposite direction.

5. The staged release aortic stent graft according to any one of claims 1 to 4, wherein a plurality of axially arranged support rods are fixed with spacing at least at the proximal end along the circumferential direction on the covering.

6. The staged release aortic stent graft according to claim 5, wherein the support rod is fixed on the outer wall or the inner lumen wall surface of the covering, and the connector is fixed on outer wall surface of covering that corresponding to the support rod, or the connector is arranged on the support rod or the covering around the support rod.

7. The staged release aortic stent graft according to claim 5, wherein the support rod is fixed on the covering by sewing, heat sealing or bonding.

8. The staged release aortic stent graft according to claim 5, wherein at least one fixing point for fixed connection with the covering is arranged on the support rod, and the fixing point is a connection hole or an opening slot with an opening arranged on the support rod.

9. The staged release aortic stent graft according to claim 5, wherein the support rods are arranged parallel to the central axis of the stent ; or the support rods are arranged in a shape of a figure eight expressed in Simplified Chinese or a shape of an inverted figure eight expressed in Simplified Chinese.

10. The staged release aortic stent graft according to any one of claims 1 to 4, wherein the stent is a tubular structure extending with an equal diameter or a non-equal diameter.

11. The staged release aortic stent graft according to claim 10, wherein a fenestration for arranging branch stents or branch blood vessels is arranged at the proximal end or middle of the stent.

12. The staged release aortic stent graft according to claim 11, wherein the connectors are arranged at both sides of the fenestration, for restraining a portion of the stent behind the fenestration.

13. The staged release aortic stent graft according to claim 10, wherein the stent is a tubular structure extending with a non-equal diameter, which comprises a main body section and an extension section, and the diameter of the main body section is greater than the diameter of the extension section, and a transition section is arranged between the main body section and the extension section; a fenestration for placing branch stents or branch blood vessels is arranged at the main body section or/and the transition section.

14. The staged release aortic stent graft according to claim 10, wherein the connector is arranged at both sides of the fenestrations of the main body section and the transition section, for restraining a portion of the stent behind the fenestration.

15. The staged release aortic stent graft according to claim 10, wherein a plurality of axially arranged support rods are fixed with spacing at least at the proximal end along the circumferential direction on the covering, and the support rod is fixed on the outer wall or the inner lumen wall surface of the covering, and the connectors are fixed on outer wall surface of covering that corresponding to the support rods, or the connectors are arranged on the support rods or the covering around the support rods.
